# FASCICULE DE BREVET EUROPEEN

(11) **EP 4 404 825 B1**
(45) Date de publication et mention de la délivrance du brevet: **02.07.2025**
(21) Numéro de dépôt: 22792845.4
(22) Date de dépôt: 22.09.2022
(51) Int. Cl.: A61B 5/00, A61B 5/145, A61B 5/1495

(54) **ENSEMBLE DE PRÉPARATION D'UN CAPTEUR D'UN DISPOSITIF DE SURVEILLANCE CORPORELLE**
ANORDNUNG ZUR VORBEREITUNG EINES SENSORS FÜR EINE KÖRPERÜBERWACHUNGSVORRICHTUNG
ASSEMBLY FOR PREPARING A SENSOR FOR A BODY MONITORING DEVICE

(30) Priorité: 22.09.2021 FR 2109966
(43) Date de publication de la demande: 31.07.2024
(73) Titulaire: WIZP AS, 3183 Horten (NO)
(72) Inventeur: PIERART, Luc, 94800 Villejuif (FR)
(74) Mandataire: Regimbeau
(86) Numéro de dépôt international: PCT/FR2022/051784
(87) Numéro de publication internationale: WO 2023/047058

(56) Documents cités:
- WO-A1-2020/025822
- US-A1- 2005 130 292
- US-A1- 2009 099 427

## Description

### DOMAINE DE L'INVENTION

L'invention concerne les dispositifs prêt-à-porter ou « wearables » utilisés dans des systèmes de surveillance corporelle, par exemple pour le relevé et le suivi de paramètres biochimiques du corps humain.

L'invention concerne notamment un ensemble de préparation d'un capteur comprenant une capsule d'un dispositif de surveillance d'un analyte corporel, la capsule comprenant un capteur à aiguille(s) configuré pour fournir une mesure de concentration d'analyte corporel, et un patch sur lequel le capteur est monté, l'ensemble permettent de préparer et éventuellement protéger le capteur.

### ETAT DE LA TECHNIQUE

La surveillance de nombreuses maladies chroniques connues chez l'humain nécessite un relevé quotidien de paramètres biochimiques. Un niveau de concentration d'un analyte corporel dans un fluide corporel de l'organisme, par exemple dans le plasma sanguin ou dans le liquide interstitiel des cellules de l'organisme, peut être relevé.

A titre d'exemple répandu, le suivi du diabète chez un patient nécessite un relevé précis quotidien de la glycémie du patient.

Une solution pour réaliser le suivi du diabète consiste à réaliser une ponction, par exemple au bout du doigt, pour faire perler une goutte de sang, puis à réaliser une mesure quotidienne de glycémie dans la goutte de sang ainsi obtenue.

Des systèmes de suivi ont été proposés pour se passer de la nécessité d'une ponction manuelle, de sorte à rendre la mesure de glycémie moins laborieuse et moins invasive. On parle de systèmes CGM, pour « Continuous Glucose Monitoring » en anglais.

Certains de ces systèmes CGM réalisent, à intervalles réguliers, une mesure de glycémie au niveau du liquide interstitiel entre les cellules de la peau. La glycémie du liquide interstitiel est très proche de la glycémie du plasma sanguin. Les mesures au niveau du liquide interstitiel permettent un suivi simple et peu invasif de la glycémie des patients ; ces mesures peuvent être réalisées à l'aide de capteurs à aiguille, en transcutané, ou de manière non invasive, comme par exemple en iontophorèse ou en implantable avec une mesure par chimio-fluorescence.

La demande internationale publiée sous le numéro WO 2018/104647 décrit un système de surveillance corporelle, utilisable notamment pour le suivi de la glycémie. Ce système de surveillance inclut une montre électronique attachable au poignet à l'aide d'un bracelet. La montre comporte un boîtier, dans lequel s'insère une capsule amovible interchangeable contenant un capteur à micro-aiguilles. Le capteur est commandé de manière automatique à l'électronique du boîtier, pour réaliser une mesure transcutanée. La mesure de glycémie par le capteur est une mesure électrochimique.

Le système de surveillance corporelle décrit dans le document susmentionné présente l'avantage significatif de fournir une mesure autonome de calibration en mesurant une concentration de référence en un analyte corporel chez l'utilisateur.

Le nombre de manipulations que l'utilisateur doit réaliser pour obtenir ses mesures quotidiennes est très fortement réduit. En particulier, l'utilisateur a besoin de réaliser peu de ponctions manuelles (par exemple une simple ponction hebdomadaire), voire plus aucune ponction manuelle. Un autre avantage de ce système est son risque hygiénique faible, car les aiguilles du capteur ne sont pas en contact avec l'environnement extérieur, une fois ces aiguilles insérées dans la peau.

De plus, la maintenance du système susmentionné est simple, car pour remplacer un capteur défaillant, il suffit de retirer la capsule amovible et d'insérer une capsule neuve.

Toutefois, il peut s'écouler un temps important - par exemple plusieurs mois - entre la production de la capsule amovible comportant le capteur à micro-aiguilles et l'insertion de la capsule amovible dans un dispositif prêt-à-porter porté par l'utilisateur. En outre, la qualité de la mesure de glycémie fournie par le capteur se détériore au cours d'une période aussi longue de non-utilisation du capteur entre la production et son utilisation.

La détérioration des performances de mesure du capteur à aiguilles s'explique par une dérive de la mesure électrochimique au cours du temps. Cette dérive peut être due à une température ambiante inadaptée dans les zones de stockage des capsules amovibles comprenant les capteurs à aiguilles. Notamment, des températures trop chaudes sont nuisibles à l'intégrité du système de mesures.

Si des variations brutales et importantes de la température du capteur se produisent, cela aggrave la dérive de la mesure électrochimique.

Une première méthode connue pour pallier cette dérive est une calibration « d'usine » réalisée à l'échelle d'un lot entier de capteurs à aiguilles, à l'issue de la production du lot. Un code alphanumérique est alors inscrit sur un emballage de la capsule amovible contenant le capteur, ou est intégré à une mémoire électronique du capteur. Le dispositif prêt-à-porter possède un abaque dans lequel chaque code alphanumérique est associé à une valeur de référence. Lors de l'insertion de la capsule dans le dispositif prêt-à-porter, le code alphanumérique est lu et la calibration peut être réalisée.

Cette calibration d'usine connue de l'état de la technique, fondée sur l'utilisation d'abaques, ne donne cependant pas entière satisfaction. Des dérives de la mesure électrochimique fournie par le capteur sont toujours observées.

Une deuxième méthode de calibration connue, parfois utilisée en combinaison avec la première, consiste dans des mesures de calibration (ponctions manuelles) réalisées régulièrement par l'utilisateur pendant qu'il porte le dispositif prêt-à-porter. Les mesures de calibration sont réalisées manuellement par l'utilisateur, par exemple de façon quotidienne ou hebdomadaire. Pour réaliser la calibration manuelle, l'utilisateur peut être amené à saisir une valeur de glycémie capillaire sur le dispositif prêt-à-porter.

Dans cette deuxième méthode, cumulable avec la première, l'utilisateur est responsable de la bonne calibration de son propre dispositif prêt-à-porter. Une opération manuelle régulière est requise, ce qui va à l'encontre de l'un des objectifs d'un système autonome de surveillance corporelle, à savoir limiter la gêne ressentie par l'utilisateur.

Au regard des considérations ci-avant, l'état de la technique ne donne pas satisfaction concernant la préparation des capteurs à aiguilles pour les dispositifs prêt-à-porter, tels que des montres électroniques à base d'aiguilles ou des patchs à base d'aiguilles. Certains systèmes connus n'assurent pas une précision durable de la mesure électrochimique, et d'autres systèmes connus nécessitent des calibrations régulières qui doivent être mises en oeuvre par l'utilisateur.

### EXPOSE DE L'INVENTION

Un objectif de l'invention est de proposer des améliorations pour un dispositif de surveillance corporelle comprenant un capteur à aiguille(s), afin que la mesure fournie par ce capteur demeure fiable et précise au cours du temps.

Notamment, on recherche une solution pour réaliser facilement la calibration de ce capteur, en limitant la gêne causée pour l'utilisateur. L'objectif est de supprimer la nécessité de ponctions manuelles quotidiennes et de passer a *minima* à des ponctions manuelles hebdomadaires, voire de supprimer la nécessité des ponctions manuelles.

En corollaire, on souhaite que l'utilisateur du dispositif de surveillance corporelle ne soit pas responsable de la bonne calibration dudit capteur, au cours du cycle de vie du système de surveillance. On souhaite éviter la nécessité de ponctionner régulièrement le doigt pour réaliser une calibration quotidienne du capteur.

La solution recherchée doit demeurer très simple d'utilisation, afin d'assurer une expérience utilisateur agréable.

En outre, tout cela doit pouvoir se faire dans conditions telle que le capteur demeure protégé jusqu'à sa destination finale.

A ce titre, l'invention concerne un ensemble comprenant une capsule d'un dispositif de surveillance d'un analyte corporel, la capsule comprenant un capteur configuré pour fournir une mesure de concentration d'analyte corporel, le capteur comprenant une surface de capteur et au moins une aiguille fixée au capteur sur la surface de capteur, la capsule comprenant en outre un patch supportant le capteur, le patch recouvrant au moins en partie la surface de capteur, une partie du patch définissant un ajour dans lequel les aiguilles du capteur sont disposées, l'ensemble étant caractérisé en ce qu'il comprend : au moins un premier espaceur compressible, présentant une première face en face du patch et une deuxième face opposée à la première face, une poche perçable disposée à distance et en face de l'aiguille, l'ensemble étant tel que lorsque l'aiguille subit une poussée en direction de la poche elle perce la poche, l'espaceur étant compressé, l'aiguille revenant en face et à distance de la poche en absence d'une quelconque poussée, l'espaceur étant décompressé.

L'invention est avantageusement complétée par les caractéristiques suivantes, prises seules ou en une quelconque de leur combinaison techniquement possible.

L'ensemble comprend une membrane rigide fixée sur la deuxième face de l'espaceur et s'étendant à distance et en face de l'aiguille, ladite membrane étant configurée pour fermer l'ajour afin de protéger l'aiguille.

La membrane rigide est formée d'un matériau élastique ou semi-élastique.

La membrane est perçable par l'aiguille, la poche étant disposée au-dessous de la membrane, l'ensemble étant configuré pour que lorsque l'aiguille subit une poussée en direction de la membrane elle perce, outre la poche, la membrane et revient complètement dans l'ajour en face et à distance de la membrane en absence d'une poussée.

La poche est disposée dans l'ajour et repose sur une surface de la membrane qui débouche dans l'ajour.

L'espaceur en position compressée ou non compressée présente une épaisseur supérieure ou égale à 0,1 millimètre et inférieure ou égale à 2 millimètres.

L'espaceur comprend au moins une première partie d'espaceur et une deuxième partie d'espaceur opposé à la première partie, la première partie et la deuxième partie étant de préférence disjointes.

Le patch comprend un film pelable entre la couche d'adhésif du patch et l'espaceur, la première face de l'espaceur étant collée fixement sur le film pelable, le film pelable étant configuré pour permettre de détacher le film pelable, l'espaceur, et éventuellement la membrane d'une couche externe du patch.

L'ensemble comprend un réceptacle, le capteur étant mobile par rapport au réceptacle entre une première position dans laquelle l'aiguille est à distance et en face de la poche, l'espaceur étant alors non compressé, et une deuxième position dans laquelle la poche est percée par l'aiguille et dans laquelle l'aiguille est au contact d'un volume de la poche, l'espaceur étant alors compressé.

L'ensemble comprend au moins un premier élément d'appui situé en face de l'espaceur, le premier élément d'appui étant configuré pour faciliter la compression de l'espaceur et le perçage de la poche par l'aiguille.

L'ensemble comprend au moins un deuxième élément d'appui situé en face de la poche, le deuxième élément d'appui étant configuré pour faciliter le perçage de la poche.

Le volume de la poche de préparation comprend une solution de calibration présentant une concentration en glucose égale à une valeur de référence supérieure ou égale à deux millimoles de glucose par litre et inférieure ou égale à 20 millimoles de glucose par litre.

Le volume de la poche de préparation comprend une solution de mouillage configurée pour humidifier des éléments actifs de l'aiguille.

L'ensemble comprend des moyens d'appui montés sur le réceptacle, les moyens d'appui étant configurés pour déplacer le capteur par rapport au réceptacle depuis la première position vers la deuxième position, l'élément d'appui jouant alors le rôle de contre-appui.

L'ensemble permet de pré-mouillée les aiguilles et de calibrer le capteur.

La membrane permet de protéger les aiguilles dans un environnement stérile tant qu'elle n'est pas percée avant son utilisation. Les aiguilles sont ainsi protégées des éventuels chocs, de la poussière etc. présents au cours de la manipulation de la capsule une fois les aiguilles mouillées.

En outre, une fois le capteur calibré sa mise en service est simple par la présence du film pelable qui libère les aiguilles pour insertion dans la peau de l'utilisateur.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre une vue d'ensemble d'un dispositif de surveillance d'un analyte corporel
- la figure 2 illustre une vue schématique d'un capteur à aiguilles selon l'invention ;
- la figure 3a et la figure 3b illustrent un ensemble de préparation d'un capteur selon un premier mode de réalisation de l'invention dans une première position et dans une deuxième position ;
- la figure 4a et la figure 4b illustrent un ensemble de préparation d'un capteur selon un deuxième mode de réalisation de l'invention dans une première position et dans une deuxième position.

Sur l'ensemble des figures, les éléments similaires portent des références identiques.

### DESCRIPTION DETAILLEE

La description ci-après se rapporte à la préparation d'un capteur à aiguille(s) intégré à une montre électronique. Le capteur est conçu pour fournir une mesure de concentration en glucose au sein du liquide interstitiel d'un porteur. La montre électronique constitue avec le capteur un dispositif de surveillance corporelle.

Par « surveillance corporelle », on entend la vérification de constantes biochimiques du porteur du système de surveillance, typiquement la concentration du liquide interstitiel du porteur en une protéine, une hormone, un biomarqueur, en oxygène, en nutriments, etc. L'homme du métier comprendra aisément que d'autres grandeurs physiques peuvent être suivies par le système de surveillance, comme par exemple la concentration en lactate, l'hydratation, etc.

Dans toute la suite, la constante biochimique à surveiller est par exemple la concentration en glucose (ou glycémie) au sein du liquide interstitiel de la peau. La glycémie du liquide interstitiel est considérée comme représentative de la glycémie du plasma sanguin. On comprendra que l'ensemble de préparation de capteur décrit ci-après peut être utilisé, avec les mêmes avantages, pour préparer un capteur destiné à mesurer un autre paramètre biochimique. En outre, l'aiguille ou les aiguilles du capteur pourraient être destinées à être insérées dans un autre fluide corporel que le liquide interstitiel, par exemple dans le sang.

En outre, le dispositif prêt-à-porter est, dans toute la description ci-après, une montre électronique configurée pour afficher des informations à l'attention de son porteur. Toutefois, l'ensemble de préparation de capteur décrit ci-après peut être utilisé, avec les mêmes avantages, en association avec tout autre type de dispositif prêt-à-porter : bracelet, tracker, patch électronique, lecteur radio électronique, etc.

### Architecture générale d'un dispositif de surveillance corporelle

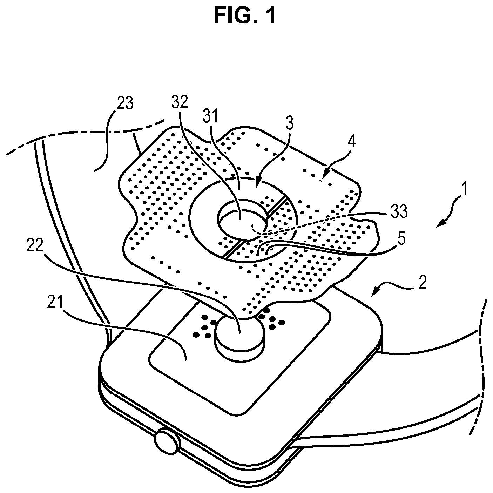

La **figure 1** illustre un dispositif 1 de surveillance corporelle comprenant un boitier 2, un capteur 3 et un patch adhésif 4.

Le capteur 3 est dans ce cas un capteur à aiguilles prévu pour fournir une mesure de courant électrique au sein du liquide interstitiel du porteur du dispositif 1.

Des aiguilles 5 sont avantageusement disposées sur une face interne 31 du capteur 3. Cette face interne 31 est destinée à être placée sur la peau du porteur.

Le capteur 3 est assemblé au patch adhésif 4 constituent ensemble une capsule. Le capteur 3 peut aussi être amovible par rapport au patch 4. Une telle capsule est avantageusement montée amovible avec le boitier 2. En particulier, la capsule et donc le capteur 3 s'engage de manière préférée dans une cavité 21 du boitier 2 située sur sa face destinée à être en contact avec la peau. Le capteur 3 comprend une face externe 32 opposée à la face interne 31.

Le boîtier 2 et la capsule peuvent présenter des formes complémentaires, ce qui limite l'effort nécessaire pour la bonne insertion de la capsule contre le boîtier 2.

Le patch 4 comporte une couche adhésive, ou est lui-même formé en un matériau adhésif. Le patch permet donc l'attache de la capsule à la peau du porteur, et favorise le maintien des aiguilles 5 dans le liquide interstitiel. Le patch 4 présente par exemple une forme annulaire, et recouvre la capsule.

Le capteur 3 ici illustré est de forme circulaire avec un orifice central 33 mais elle peut prendre d'autres formes : rectangulaire, oblongue, ellipsoïdale avec ou sans orifice central. L'orifice central 33 permet de positionner correctement le capteur 3 dans la cavité 21 du boitier qui comprend un plot central de positionnement (non représenté).

Le capteur 3 comprend donc des éléments qui permettent de prélever le liquide soit d'amener les signaux détectés par chaque microaiguille vers le boitier 2 pour traitement (non décrit ici).

Le capteur 3 peut prendre la forme d'une plaque de plastique, d'un circuit imprimé (rigide ou flexible en silicium), d'une plaque en métal non conducteur comme par exemple de l'aluminium.

Le patch adhésif 4 est adapté pour être collé à la peau et supporte le capteur 3 et permet de détacher le boitier 2 sans enlever le capteur 3 en le gardant collée au corps. Une telle configuration permet d'éviter d'enlever le capteur pour certaines opérations qui n'implique que le boitier : rechargement de la batterie, réparation, remplacement, extraction des données vers un ordinateur.

Le boitier 2 est avantageusement en forme de boitier de montre et comprend des moyens d'attaches 23 du dispositif au poignet d'un utilisateur. Il s'agit notamment d'un bracelet adapté pour entourer le poignet d'un utilisateur. Le bracelet est de préférence réglable.

Le boitier 2 loge plusieurs éléments permettant d'analyser ou d'extraire du liquide interstitiel. A ce titre, on pourra se référer au document WO 2019/141743 au nom du déposant qui décrit en détail la mesure et la détection d'une grandeur physique à partir de microaiguilles au contact d'un liquide corporel pouvant être prélevé ou non.

Avantageusement, la montre comprend en outre une interface de communication sans fil, par exemple via un réseau de télécommunications de type 3G et/ou 4G et/ou 5G et/ou Wi-Fi et/ou Bluetooth et/ou NFC et/ou DECT.

Également, la montre peut comprendre un indicateur lumineux tel qu'une diode, qui peut être utilisé pour signaler la fin d'une opération de préparation du capteur.

Les aiguilles 5 sont avantageusement des micro-aiguilles. Le capteur 3 comporte de préférence entre quatre et cinquante micro-aiguilles voire quatre cent microaiguilles. Bien entendu, un nombre différent peut être considéré sans que cela ne limite la description de l'invention ici faite.

On entend par micro-aiguille, une aiguille présentant une hauteur faible de préférence entre 10 µm et 1000 µm, de préférence entre 0,3 mm et 0,8mm. La hauteur des micro-aiguilles est suffisamment faible pour éviter le contact avec un nerf de douleur mécanique du porteur lorsque le dispositif est porté.

Les microaiguilles 5 permettent de mesurer ou prélever le liquide corporel.

Les microaiguilles 5 sont creuses lorsqu'il s'agit de prélever du liquide soit pleine pour analyser directement le liquide. Lorsqu'il s'agit de prélever le liquide, les microaiguilles permettent l'extraction de liquide interstitiel du derme de façon indolore sans perlement de sang et l'envoie vers un capteur logé dans le boitier 2. Lorsqu'il s'agit d'analyser du liquide, les microaiguilles ne prélèvent pas de liquide et intègrent le capteur sur leur surface sous la forme d'un revêtement tel qu'un matériau biochimique apte à réagir avec l'analyse à effectuer sur le liquide.

La longueur des aiguilles 5 est ainsi suffisamment réduite pour éviter le contact avec un nerf de l'utilisateur, pour limiter la douleur causée par le port du dispositif 1.

Chaque aiguille présente par exemple une forme pyramidale.

Dans le présent exemple, chaque aiguille 5 comprend à sa surface au moins un matériau chimique ou biochimique apte à réagir avec l'analyte corporel dont on souhaite obtenir une mesure (c'est-à-dire ici le glucose). Un matériau apte à réagir avec l'analyte corporel est par exemple une enzyme capable d'oxyder l'analyte corporel.

Dans un exemple alternatif, chaque aiguille 5 comprend une cavité interne située à l'arrière de la pointe, et le matériau chimique ou biochimique apte à réagir avec l'analyte se trouve dans cette cavité interne.

Dans un autre exemple alternatif, le capteur 3 peut comprendre des cavités situées à l'arrière des aiguilles 5, et/ou à l'intérieur des aiguilles 5. Par exemple, une ou plusieurs aiguilles 5 peuvent comprendre un canal ouvert. Les cavités comprennent le matériau chimique ou biochimique apte à réagir avec l'analyte. Les aiguilles 5 sont alors aptes à faire remonter le fluide corporel jusqu'auxdites cavités.

De manière avantageuse, le capteur 3 comprend plusieurs microaiguilles qui consistent en un réseau de microaiguilles en ce qu'elles sont électriquement connectées entre elles par groupe. Les microaiguilles transpercent la peau pour venir au contact du liquide interstitiel lorsque le capteur est au contact de la peau.

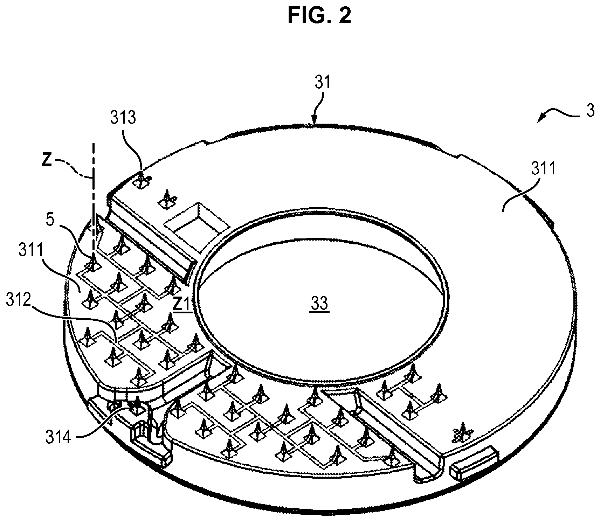

Le capteur 3 illustré sur la figure 2 comprend, en complément des aiguilles 5, un substrat 311 doté d'une pluralité de pistes métalliques 312, une électrode de conductivité de travail 313, une électrode de conductivité de référence 314.

En cours d'utilisation du capteur 3 pour réaliser une mesure, une tension est générée entre plusieurs aiguilles. Au moins une partie des aiguilles 5 du capteur 3 sont au moins partiellement immergées dans le liquide interstitiel. Le matériau chimique ou biochimique présent à la surface des aiguilles 5 réagit avec le glucose du liquide interstitiel.

Le capteur 3 fournit ainsi une mesure de courant électrique, représentative de la concentration en glucose dans le liquide interstitiel.

Le substrat 311 et les aiguilles 5 sont préférentiellement disposés sur une unique face du capteur 3, qui est la face dirigée vers le haut selon l'orientation de la figure 2. Cette face haute est destinée à être disposée face à la peau de l'utilisateur.

Chaque aiguille s'étend de la face haute selon une direction Z, depuis sa base jusqu'à sa pointe. La direction Z est de préférence orthogonale à un plan de la face haute.

L'ouverture 33 centrale est ici de forme circulaire. Le capteur 3 présente ainsi, dans le présent exemple, une forme générale annulaire.

Pour un exemple détaillé de structure du capteur 3, on pourra se référer à la demande internationale publiée sous le numéro WO 2020/025822 et notamment à la description afférente aux Figures 1 et 2 de ce document.

Un support du capteur 3 (par exemple la capsule amovible) comprend de préférence un patch (non illustré) attachable à la peau de l'utilisateur.

Le capteur 3 est ici prévu pour être commandé par l'unité de traitement 14 de la montre 10.

Dans une variante possible, le capteur 3 comporte une mémoire, configurée entre autres pour enregistrer une information de recalage. L'information de recalage peut alors être enregistrée dans le capteur 3 après transmission par une unité de traitement intégrée à la montre 10, au cours d'un procédé de calibration du capteur 30.

### Ensemble : capsule et poche

On décrit dans ce qui suit un ensemble comprenant une capsule et une poche en vue de « préparer » le capteur avant son utilisation et notamment sa mise en place dans le boitier 2.

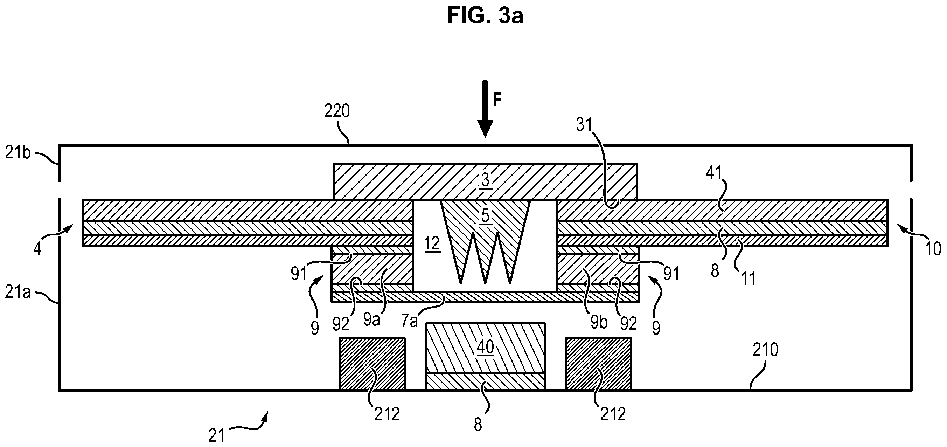

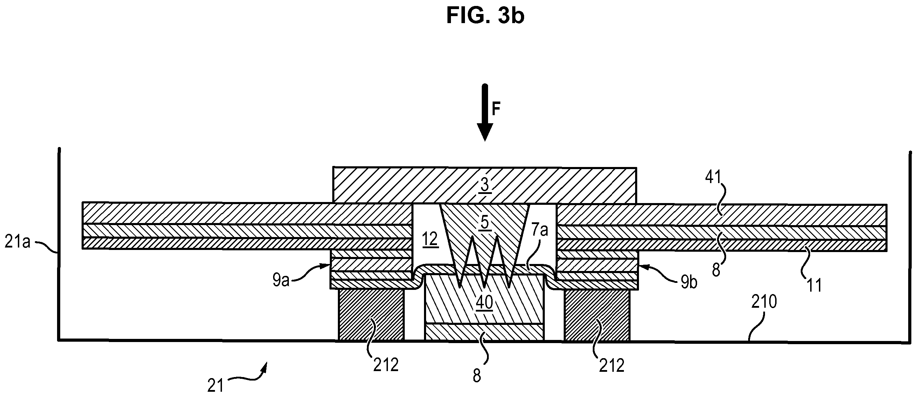

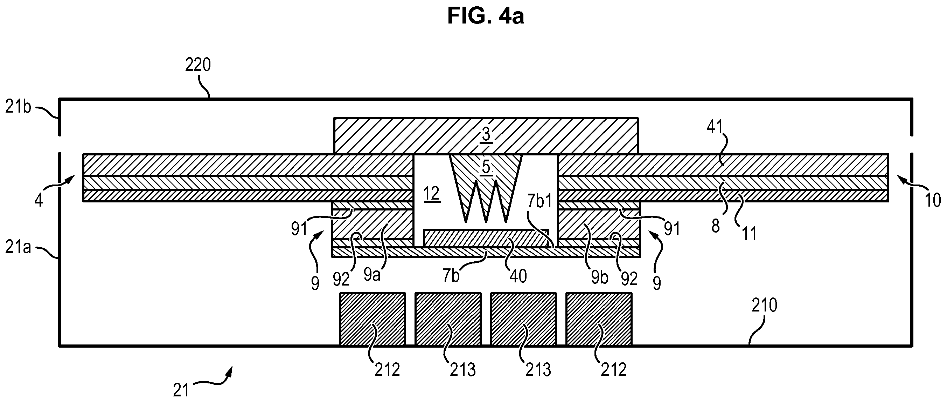

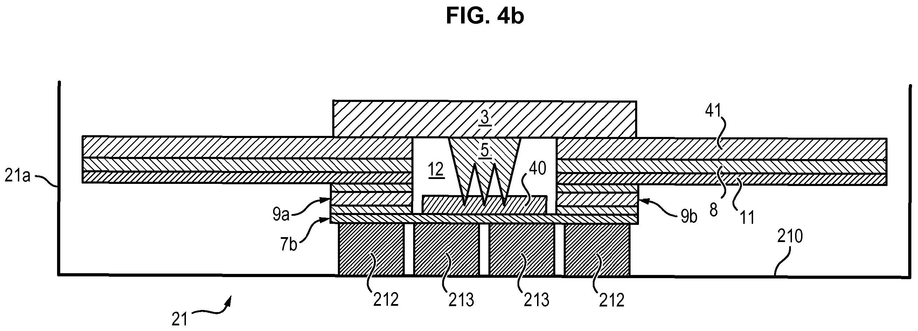

Les **figures 3a****,** **3b** illustrent un ensemble comprenant une capsule 10 et une poche 40 selon un premier mode de réalisation présenté selon une première position et une deuxième position. Les **figures 4a****,** **4b** illustrent un ensemble comprenant une capsule 10 et une poche 40 selon un deuxième mode de réalisation présenté selon une première position et une deuxième position.

En particulier, et comme déjà indiqué, la capsule 10 comprend outre le capteur 3, le patch 4 auquel le capteur 3 est attaché, le patch 4 recouvrant au moins en partie la surface de capteur 3. Une partie, par exemple centrale du patch 4 définit un ajour 12 dans lequel les aiguilles 5 du capteur 3 sont disposées. On considère une surface du capteur 5 ici la surface interne 31 du capteur 5 qui est dirigée vers la peau du porteur. Sur les figures 3a, 3b et 4a, 4b le patch 4 est au-dessous du capteur 3, toutefois le patch 4 peut être au-dessus du capteur 3.

L'ajour 12 présente, de préférence, selon une direction diamétrale une dimension minimale, supérieure ou égale à 2 millimètres, de préférence supérieure ou égale à 5 millimètres. L'ajour 12 définit donc un espace libre dans lequel les aiguilles 5 sont disposées. L'ajour 12 dans lequel sont positionnées les aiguilles constitue donc avantageusement une cavité qui permet de protéger les aiguilles 5 notamment si elles sont amenées à être manipulés en vue de l'installation du capteur 3 dans le boitier 2.

L'ajour 12 est avantageusement fermé dans sa partie inférieure par une membrane 7a, 7b rigide qui peut être perçable. Nous y reviendrons. La partie inférieure de l'ajour 12 est celle qui est débouchante. La membrane 7a, 7b permet de fermer la partie ouverte de l'ajour 12 et permet donc de protéger les aiguilles 5 dans l'enceinte formée par l'ajour 12 et la membrane 7a, 7b.

On entend par membrane une couche rigide de matériau(x) qui présente une épaisseur fine supérieure ou égale à 0,05 mm et inférieure ou égale à 0,50 mm.

La membrane 7a, 7b rigide est formée d'une couche en matériau élastique ou semi-élastique. Par exemple, la membrane est constituée en un maillage de fils fins, une épaisseur moyenne du maillage étant de préférence supérieure ou égale à 0,05 millimètre et inférieure ou égale à 0,50 mm.

Alternativement, la membrane est en caoutchouc ou en peau artificielle ou en métal ou en papier. Bien entendu d'autres matériaux ou combinaison de matériaux sont possibles.

Au moins un espaceur 9 compressible présentant une première face 91 et une deuxième face 92 opposée à la première face 91 est attaché au patch 4 au niveau de l'ajour 12.

En outre, une poche 40 perçable est disposée à distance et en face de la ou les aiguille(s) 5.

Selon cette configuration, lorsque la ou les aiguilles subissent une poussée en direction de la poche 40 elles peuvent percer la poche 40 et a contrario sortir de la poche en absence de la poussée F (voir les figures 3b et 4b sur lesquelles la poche 40 est percée).

En particulier et de manière complémentaire, l'espaceur 9 est configuré pour se décompresser une fois la poussée F relâchée, de sorte que l'aiguille 4 retrouve une position à distance de la poche dans l'ajour 12.

C'est le caractère compressible de l'espaceur 9 (qui peut se compresser) et qui permet donc aux aiguilles de venir percer la poche 40.

L'espaceur 9 est donc dimensionné et configuré pour notamment venir s'appuyer sur un premier élément d'appui 212 en face et se comprimer sur ce premier élément d'appui 212. Ce premier élément d'appui 212 joue le rôle de contre-appui pour l'espaceur 9.

De manière avantageuse, l'espaceur 9 présente une épaisseur moyenne en position compressée ou non compressée qui est supérieure ou égale à 0,1 millimètre et est inférieure ou égale à 2 millimètres.

En outre, l'épaisseur de l'espaceur 9 est choisie en fonction de la hauteur des aiguilles 5 et de l'épaisseur du patch 4.

De manière préféré, l'espaceur 9 est en un matériau élastique ou semi-élastique et est par exemple une mousse ayant des propriétés élastiques. Il peut aussi s'agir par exemple de caoutchouc ou d'une lame ressort.

De manière préférée, l'espaceur 9 comprend au moins une première partie d'espaceur 9a et une deuxième partie d'espaceur 9b, l'espaceur 9 étant formé de plusieurs pièces. De manière complémentaire, la première partie 9a et la deuxième partie 9b sont disjointes. Alternativement, la première partie et la deuxième partie sont d'une pièce. En revanche, lorsque l'espaceur est constitué de parties disjointes celles-ci peuvent être au nombre 3, 4 voire plus. Dans tous les cas l'espaceur entoure les aiguilles 5 au niveau de l'ajour 12 comme c'est visible sur les figures 3a, 3b, 4a, 4b.

La poussée F nécessaire pour percer la poche 40 est supérieure ou égale à 10 Newton et inférieure ou égale à 50 Newton, de préférence égale à 10 Newton. Sachant que 10 Newton correspond à 1kg d'appui cela correspond à ce que peut faire aisément une personne âgée ou un enfant. En d'autres termes cela couvre un grand nombre d'utilisateurs. L'ensemble poche 40 et espaceur 9 sont donc configurés de cette manière.

Le patch 5 comprend différentes couches d'adhésif 8 permettant de fixer le capteur 3 à la couche externe 41 du patch 4. Des couches d'adhésifs permettent aussi de fixer l'espaceur 9 à un film pelable 11 et de fixer la membrane 7a, 7b à l'espaceur 9 sur son autre face 92. L'idée ici est de pouvoir détacher simplement en enlevant le film pelable 11 de la couche externe 41 du patch 4, celle qui restera après la mise en place du capteur 3, l'espaceur 9 et la membrane 7a, 7b. On précise que l'on entend par matériau pelable un revêtement de surface qui peut être détaché par voie sèche et sans rupture.

Un réceptacle 21 loge les éléments ci-dessus et comprend notamment une surface de réception 210 sur laquelle est disposée l'élément d'appui 212 en face de l'espaceur 9 afin d'en faciliter sa compression. Un tel élément d'appui 212 prend la forme d'une bosse faisant saillie de la surface de réception 210 du réceptacle 21, la bosse 212 étant placées en face de l'espaceur 9. Dans le cas où il y a plusieurs espaceurs on prévoit autant de bosses que d'espaceurs.

La surface de réception 210 est dans la partie inférieure 21a du réceptacle 21.

Une partie supérieure 21b du réceptacle forme un couvercle et se retire pouvoir positionner la capsule au boitier 2.

Le réceptacle 21 comprenant tous les éléments décrits ci-dessus et constitue une enceinte de stockage stérile de la capsule en vue de préparer le capteur 3.

Ainsi, le réceptacle 21 est de forme générale parallélépipédique et prend la forme d'une boite à l'intérieur de laquelle sont placés le capteur 3, le patch 4 et la poche 40. Alternativement, le réceptacle 21 peut être un dock de charge, etc.

La membrane 7a, 7b protège donc les aiguilles du capteur 3 contre les contaminations et vibrations alors que le capteur 3 est sur le patch 4, et permet en combinaison avec la poche de préparation de calibrer ou de pré-mouiller les aiguilles quand le capteur est poussé vers la poche.

En fonctionnement, le capteur 3 est mobile par rapport au réceptacle 21 entre une première position dans laquelle l'aiguille 5 est à distance et en face de la membrane 7a, 7b et de la poche 40, l'espaceur 9 étant alors non compressé, et une deuxième position dans laquelle la membrane 7a, 7b et la poche 40 sont percés par l'aiguille 5 et dans laquelle l'aiguille 5 est au contact d'un volume de la poche de préparation 40, l'espaceur 9 étant alors compressé.

A ce titre, l'ensemble comprend des moyens d'appui montés sur le réceptacle 21, les moyens d'appui étant configurés pour déplacer le capteur 2 par rapport au réceptacle 21 depuis la première position vers la deuxième position, la bosse 212 jouant le rôle de contre-appui pour l'espaceur 9. Les moyens d'appui sont le couvercle 21b du réceptacle 21 ou bien le boitier 2, le capteur 3 étant alors assemblé au boitier 2.

### Poche de préparation

La poche 40 (ou poche de préparation) comprend un volume d'une solution de préparation du capteur.

Ici, la solution de préparation est une solution de calibration, présentant une concentration volumique en un analyte corporel égale à une valeur de référence prédéterminée.

On rappelle que, dans le présent exemple, l'analyte corporel à analyser est le glucose, et la mesure fournie par le capteur 3 est représentative de la glycémie.

Ainsi, la solution contenue dans la poche 40 présente ici une concentration en glucose connue de manière précise. Cette concentration est avantageusement comprise entre 2 millimoles de glucose par litre et 20 millimoles de glucose par litre, plus préférentiellement entre 6 millimoles de glucose par litre et 10 millimoles de glucose par litre.

De manière alternative, la solution de préparation contenue dans la poche 40 peut être une solution de pré-mouillage, c'est-à-dire une solution qui reproduit la nature chimique du fluide corporel dans lequel le capteur 3 est destiné à être inséré (à savoir le liquide interstitiel ici). Le pré-mouillage consiste à humidifier une partie des matériaux chimiques ou biochimiques (tels que des éléments enzymatiques actifs) qui permettent la mesure électrochimique, contenus par exemple sur les couches supérieures des aiguilles 32. Ces éléments chimiques comportent typiquement des éléments enzymatiques actifs. Grâce au pré-mouillage, on accélère le passage ultérieur des éléments électriques entre l'ensemble des couches chimiques et le fluide corporel humain (par exemple dans le liquide interstitiel), une fois le capteur à aiguilles mis en place.

Un avantage de réaliser un pré-mouillage du capteur 3, au cours d'une préparation du capteur 3, est d'accélérer la convergence des mesures ultérieures du capteur dans le fluide corporel. Le capteur 3 atteint ainsi rapidement un état opérationnel.

La solution de préparation est ici réalisée sous forme d'un gel.

En alternative, la solution de préparation peut être une solution liquide.

On notera que la poche 40 peut contenir une solution qui sert à la fois, entre autres utilisations possibles, de solution de calibration et de solution de pré-mouillage.

Quelle que soit la forme choisie pour la solution de préparation contenue dans la poche 40, la poche comprend de préférence en outre une enveloppe qui entoure le volume de solution de préparation, pour permettre le transport et la manipulation de la poche 26.

La poche 40 est perçable, de sorte qu'une aiguille 5 du capteur 3 peut atteindre la solution de préparation. Si une enveloppe est présente, celle-ci est au moins en partie perçable.

### Premier mode de réalisation : poche sur un réceptacle

On revient en détail aux figures 3a et 3b qui illustrent un ensemble de préparation selon un premier mode de réalisation.

Selon ce premier mode de réalisation, la poche 40 repose sur la surface de réception 210. Cette surface de réception 210 doit être suffisamment rigide pour maintenir la poche 40. La poche 40 est au-dessous de la membrane 7a. En outre, cette surface de réception 210 joue le rôle de contre appui pour permettre de venir percer la poche 40.

Également, l'ensemble selon ce premier mode de réalisation comprend la membrane 7a qui outre le fait d'être rigide est ici perçable et est fixée sur la deuxième face 92 de l'espaceur 9 et s'étendant à distance et en face des aiguilles l'aiguilles 5.

La membrane 7a est perçable et l'ensemble est tel que lorsque les aiguilles 5 subissent une poussée F en direction de la membrane 7a elles puissent percer la membrane 7a. A contrario, lorsque les aiguilles 5 ne subissent pas de poussée, les aiguilles reviennent dans l'ajour 12 au-dessus de la membrane 7.

On précise que l'on entend par rigide en ce que la membrane permet d'opposer une force de résistance à la pénétration par une aiguille, la membrane pouvant être percé lorsque la poussée est supérieure à cette force de résistance.

La membrane 7a est donc telle qu'elle permet d'assurer que les aiguilles reviennent dans l'ajour 12 au-dessus de la membrane 7a lorsqu'aucune poussée n'est appliquée sur les aiguilles 5.

De manière avantageuse, la membrane 7a doit être telle qu'elle ne s'accroche pas à l'aiguille 5 en l'absence de poussée pour permettre un retour des aiguilles bien au-dessus de la membrane 7a.

De manière avantageuse, pour percer la membrane 7a et la poche 40 une force de poussée nécessaire pour percer la membrane 7a est supérieure ou égale à 1 Newton et inférieure ou égale à 50 Newton, de préférence égale à 10 Newton. Ici encore, une telle force peut être déployée par un grand nombre d'utilisateurs.

La membrane 7a est fixée ici à l'espaceur 9 par l'intermédiaire d'une couche d'adhésif 8 et peut être retirée lorsque le film pelable 11 est détaché de la couche externe 41 du patch 4.

En utilisation, un utilisateur dispose de l'ensemble tel qu'illustré sur la figure 3a et désire installer un capteur 3 dans le boitier 2, l'ensemble étant « neuf ».

L'utilisateur se saisit de l'ensemble et ouvre la partie supérieure 21b du réceptacle. La capsule 10 est toujours dans le réceptacle 21 et l'utilisateur amène le boitier 2 pour le connecter au capteur 3 via sa face externe 32.

Ensuite, une poussée F est exercée le capteur 3 par l'intermédiaire du boitier 2. Ceci a pour effet que les aiguilles percent la membrane 7 et la poche 40 comme c'est visible sur la figure 3b, la membrane 7 se déformant au cours de l'opération et l'espaceur 9 se compresse sur les éléments 212. La pré-calibration nécessaire au début du cycle d'utilisateur du capteur 3 est donc effectuée, le capteur 3 étant alimentée par l'intermédiaire du boitier 2 (non décrit en détail). A ce titre, la pré-calibration corrige une dérive éventuelle de la mesure du courant électrique fournie par les aiguilles 5 du capteur 3. Les aiguilles 5 sont immergées dans un volume d'une solution de calibration contenue dans la poche 40. La concentration en analyte corporel (ici le glucose) de la solution de calibration étant connue, la valeur de la mesure de courant électrique attendue est également connue. Une correction de la mesure de courant électrique ou correction de la tension délivrée est alors effectuée en fonction de la valeur de la mesure de courant électrique attendue.

Il est à noter que la préparation du capteur 3 ne comporte pas nécessairement une calibration d'une mesure fournie par le capteur 3. La préparation du capteur 3 peut comporter uniquement un mouillage de la ou les aiguille(s), en particulier un pré-mouillage pour accélérer la convergence ultérieure de la mesure fournie par le capteur 3.

La préparation du capteur 3 peut aussi comporter en combinaison un pré-mouillage et une pré-calibration, l'ensemble de l'invention pouvant permettre les deux.

Une fois les aiguilles ainsi trempées dans la poche, la force de poussée est relâchée et les aiguilles sortent de la poche 40 et repassent par la membrane 7 pour revenir dans l'ajour 12. L'ensemble revient alors dans la position illustrée sur la figure 3a.

L'utilisateur se saisit de la capsule 10 qui comprend le capteur 3 et le patch 4 et aussi la membrane 7a. La capsule est donc à ce moment à l'air libre.

Pendant toute cette phase, les aiguilles 5 sont protégées par la membrane 7a.

Ensuite, l'utilisateur positionne la capsule 10 autour de son poignet et retire ensuite le film pelable 11 qui au cours de son retrait amène avec lui la membrane 7a, l'espaceur 9 et permet à l'utilisateur de faire adhérer le patch 4 à sa peau, la couche d'adhésif 8 directement en contact avec la couche externe 41 du patch 4 étant libérée.

### Deuxième mode de réalisation : poche sur membrane

On revient en détail aux figures 4a et 4b qui illustrent un ensemble selon un deuxième mode de réalisation.

L'ensemble selon ce deuxième mode de réalisation reprend tous les éléments du premier mode de réalisation à la différence que la poche 40 est ici disposée sur la membrane 7b. Cette membrane 7b n'a pas vocation à être percée par les aiguilles de sorte que seule la propriété de rigidité est recherchée afin de supporter la poche 40.

En revanche, la poche 40 est perçable par les aiguilles 5.

Selon ce deuxième mode de réalisation, au moins un deuxième élément d'appui 213 est disposé en dessous de la poche 40. Comme illustré, sur la figure 4a, il s'agit de deux bosses 213 faisant saillie de la surface de réception 210. Ces deuxièmes éléments d'appuis servent à faciliter le perçage de la poche 40 par les aiguilles puisqu'en fonctionnement, la membrane et la poche vont venir s'appuyer sur ces éléments d'appuis, l'espaceur 9 s'appuyant comme sur les premiers éléments d'appui 212 comme décrit précédemment.

Le fonctionnement de l'ensemble selon le deuxième mode de réalisation est similaire à celui du premier mode de réalisation à la différence qu'au retrait du film pelable la poche est aussi retirée.

La figure 4b illustre l'ensemble selon le deuxième mode de réalisation avec la poche 40 percée par les aiguilles 5, la membrane 7 vient se positionner sur les éléments d'appui en dessous, et l'espaceur 9 se compresse sur les premiers éléments d'appui 212. De cette manière on peut procéder à la calibration des aiguilles 5 comme décrit précédemment, l'ensemble revenant à la position de la figure 4a à l'issue de l'opération.

## Revendications

1. Ensemble comprenant une capsule (10) d'un dispositif (1) prêt-à-porter de surveillance d'un analyte corporel, la capsule (10) comprenant un capteur (3) configuré pour fournir une mesure de concentration d'analyte corporel, le capteur (3) comprenant une surface (31) de capteur (3) et au moins une aiguille (5) fixée au capteur (3) sur la surface (31) de capteur (3),
la capsule (10) comprenant en outre un patch (4) supportant le capteur (3), le patch (4) recouvrant au moins en partie la surface (31) de capteur (3), une partie du patch (4) définissant un ajour (12) dans lequel les aiguilles (5) du capteur (3) sont disposées,
l'ensemble étant **caractérisé en ce qu'**il comprend :
- au moins un premier espaceur (9) compressible, présentant une première face (91) en face du patch (4) et une deuxième face (92) opposée à la première face (91),
- un réceptacle (21) comprenant une surface de réception (210) ;
- une poche (40) perçable disposée sur la surface de réception (210) à distance et en face de l'aiguille (5), l'ensemble étant tel que lorsque l'aiguille (5) subit une poussée (F) en direction de la poche (40) elle perce la poche (40), l'espaceur (9) étant compressé, l'aiguille (5) revenant en face et à distance de la poche (40) en absence d'une quelconque poussée (F), l'espaceur (9) étant décompressé, le capteur (3) étant mobile par rapport au réceptacle (21) entre une première position dans laquelle l'aiguille (5) est à distance et en face de la poche (40), l'espaceur (9) étant alors non compressé, et une deuxième position dans laquelle la poche (40) est percée par l'aiguille (5) et dans laquelle l'aiguille (5) est au contact d'un volume de la poche (40), l'espaceur (9) étant alors compressé, la poche (40) comprenant un volume d'une solution de préparation du capteur (3).

2. Ensemble selon la revendication 1, comprenant une membrane (7a, 7b) rigide fixée sur la deuxième face (91) de l'espaceur et s'étendant à distance et en face de l'aiguille (5), ladite membrane (7a, 7b) étant configurée pour fermer l'ajour (12) afin de protéger l'aiguille (5).

3. Ensemble selon la revendication 2, dans lequel la membrane (7a, 7b) rigide est formée d'un matériau élastique ou semi-élastique.

4. Ensemble selon l'une quelconque des revendications précédentes, dans lequel la membrane (7a) est perçable par l'aiguille (5), la poche (40) étant disposée au-dessous de la membrane (7a), l'ensemble étant configuré pour que lorsque l'aiguille (5) subit une poussée (F) en direction de la membrane (7a) elle perce, outre la poche (40), la membrane (7a) et revient complètement dans l'ajour (12) en face et à distance de la membrane (7a) en absence d'une poussée (F).

5. Ensemble selon l'une quelconque des revendications 1 à 3, dans lequel la poche (40) est disposée dans l'ajour (12) et repose sur une surface (7b1) de la membrane (7b) qui débouche dans l'ajour (12).

6. Ensemble selon l'une quelconque des revendications 1 à 5, dans lequel l'espaceur (9) en position compressée ou non compressée présente une épaisseur supérieure ou égale à 0,1 millimètre et inférieure ou égale à 2 millimètres.

7. Ensemble selon l'une quelconque des revendications 1 à 6, dans lequel l'espaceur (9) comprend au moins une première partie d'espaceur (9a) et une deuxième partie d'espaceur (9b) opposé à la première partie (9a), la première partie (9a) et la deuxième partie (9a) étant de préférence disjointes.

8. Ensemble selon l'une quelconque des revendications 1 à 7 ou des revendications 2 à 7, dans lequel le patch (4) comprend un film pelable (11) entre une couche d'adhésif (8) du patch (4) et l'espaceur (9), la première face (91) de l'espaceur (9) étant collée fixement sur le film pelable (11), le film pelable (11) étant configuré pour permettre de détacher le film pelable (11), l'espaceur (9), et éventuellement la membrane d'une couche externe (41) du patch (4).

9. Ensemble selon l'une quelconque des revendications 1 à 8, comprenant au moins un premier élément d'appui (212) situé en face de l'espaceur (9), le premier élément d'appui étant configuré pour faciliter la compression de l'espaceur (9) et le perçage de la poche (40) par l'aiguille (5).

10. Ensemble selon l'une quelconque des revendications 1 à 8, comprenant au moins un deuxième élément d'appui (210, 213) situé en face de la poche (40), le deuxième élément d'appui étant configuré pour faciliter le perçage de la poche (40).

## Patentansprüche

1. Anordnung, umfassend eine Kapsel (10) einer tragbaren Vorrichtung (1) zur Überwachung eines Körperanalyten, wobei die Kapsel (10) einen Sensor (3) umfasst, der ausgelegt ist, um eine Messung der Konzentration des Körperanalyten bereitzustellen, wobei der Sensor (3) eine Oberfläche (31) des Sensors (3) und mindestens eine an dem Sensor (3) an der Oberfläche (31) des Sensors (3) befestigte Nadel (5) umfasst,
wobei die Kapsel (10) ferner ein den Sensor (3) tragendes Patch (4) umfasst, wobei das Patch (4) die Oberfläche (31) des Sensors (3) mindestens teilweise bedeckt, wobei ein Teil des Patchs (4) einen Durchbruch (12) definiert, in dem die Nadeln (5) des Sensors (3) angeordnet sind,
wobei die Anordnung **dadurch gekennzeichnet ist, dass** sie umfasst:
- mindestens einen ersten komprimierbaren Abstandshalter (9), der eine erste Fläche (91) gegenüber dem Patch (4) und eine zweite Fläche (92) gegenüber der ersten Fläche (91) aufweist,
- einen Behälter (21), der eine Empfangsoberfläche (210) aufweist;
- einen durchstechbaren Beutel (40), der auf der Empfangsoberfläche (210) beabstandet und gegenüber der Nadel (5) angeordnet ist, wobei die Anordnung derart ist, dass, wenn die Nadel (5) einen Schub (F) in Richtung des Beutels (40) erfährt, sie den Beutel (40) durchsticht, wobei der Abstandshalter (9) komprimiert wird, die Nadel (5) in Abwesenheit eines Schubs (F) gegenüber und beabstandet zum Beutel (40) zurückkehrt, wobei der Abstandshalter (9) dekomprimiert wird, wobei der Sensor (3) in Bezug auf den Behälter (21) zwischen einer ersten Position, in der sich die Nadel (5) in einem Abstand und gegenüber dem Beutel (40) befindet, wobei der Abstandshalter (9) dann nicht komprimiert ist, und einer zweiten Position, in der der Beutel (40) von der Nadel (5) durchstochen wird und in der die Nadel (5) mit einem Volumen des Beutels (40) in Kontakt ist, wobei der Abstandshalter (9) dann komprimiert ist, beweglich ist, wobei der Beutel (40) ein Volumen einer Zubereitungslösung des Sensors (3) umfasst.

2. Anordnung nach Anspruch 1, umfassend eine feste Membran (7a, 7b), die an der zweiten Fläche (91) des Abstandshalters befestigt ist und sich beabstandet und gegenüber der Nadel (5) erstreckt, wobei die Membran (7a, 7b) ausgelegt ist, um den Durchbruch (12) zu verschließen, um die Nadel (5) zu schützen.

3. Anordnung nach Anspruch 2, wobei die feste Membran (7a, 7b) aus einem elastischen oder halbelastischen Material besteht.

4. Anordnung nach einem der vorhergehenden Ansprüche, wobei die Membran (7a) von der Nadel (5) durchstechbar ist, wobei der Beutel (40) unterhalb der Membran (7a) angeordnet ist, wobei die Anordnung ausgelegt ist, damit, wenn die Nadel (5) einen Schub (F) in Richtung der Membran (7a) erfährt, sie außer dem Beutel (40) die Membran (7a) durchstößt und vollständig in den Durchbruch (12) gegenüber und beabstandet von der Membran (7a) zurückkehrt, wenn kein Schub (F) vorhanden ist.

5. Anordnung nach einem der Ansprüche 1 bis 3, wobei der Beutel (40) in dem Durchbruch (12) angeordnet ist und auf einer Oberfläche (7b1) der Membran (7b) aufliegt, die in den Durchbruch (12) mündet.

6. Anordnung nach einem der Ansprüche 1 bis 5, wobei der Abstandshalter (9) in komprimierter oder nicht komprimierter Position eine Dicke von größer oder gleich 0,1 Millimeter und kleiner oder gleich 2 Millimeter aufweist.

7. Anordnung nach einem der Ansprüche 1 bis 6, wobei der Abstandshalter (9) mindestens einen ersten Abstandshalterteil (9a) und einen zweiten Abstandshalterteil (9b) umfasst, der dem ersten Teil (9a) gegenüberliegt, wobei der erste Teil (9a) und der zweite Teil (9b) vorzugsweise unzusammenhängend sind.

8. Anordnung nach einem der Ansprüche 1 bis 7 oder der Ansprüche 2 bis 7, wobei das Patch (4) eine abziehbare Folie (11) zwischen einer Klebeschicht (8) des Patchs (4) und dem Abstandshalter (9) umfasst, wobei die erste Fläche (91) des Abstandshalters (9) fest auf der abziehbaren Folie (11) klebt, wobei die abziehbare Folie (11) ausgelegt ist, um das Lösen der abziehbaren Folie (11), des Abstandshalters (9) und gegebenenfalls der Membran von einer Außenschicht (41) des Patchs (4) zu ermöglichen.

9. Anordnung nach einem der Ansprüche 1 bis 8, umfassend mindestens ein erstes Stützelement (212), das sich gegenüber dem Abstandshalter (9) befindet, wobei das erste Stützelement ausgelegt ist, um das Komprimieren des Abstandshalters (9) und das Durchstechen des Beutels (40) mittels der Nadel (5) zu erleichtern.

10. Anordnung nach einem der Ansprüche 1 bis 8, umfassend mindestens ein zweites Stützelement (210, 213), das sich gegenüber dem Beutel (40) befindet, wobei das zweite Stützelement ausgelegt ist, um das Durchstechen des Beutels (40) zu erleichtern.

## Claims

1. Assembly comprising a capsule (10) of a ready-to-wear device (1) for monitoring a body analyte, the capsule (10) comprising a sensor (3) configured to provide a measure of body analyte concentration, the sensor (3) comprising a sensor (3) surface (31) and at least one needle (5) attached to the sensor (3) on the sensor (3) surface (31),
the capsule (10) further comprising a patch (4) supporting the sensor (3), the patch (4) covering at least part of the surface (31) of the sensor (3), part of the patch (4) defining an aperture (12) in which the needles (5) of the sensor (3) are arranged,
the assembly being **characterized in** it comprises:
- at least one first compressible spacer (9), with a first face (91) opposite the patch (4) and a second face (92) opposite the first face (91),
- a receptacle (21) comprising a receiving surface (210);
- a pierceable pouch (40) arranged on the receiving surface (210) at a distance from and opposite the needle (5), the assembly being such that when the needle (5) is being pushed (F) in the direction of the pouch (40), it pierces the pouch (40), the spacer (9) being compressed, the needle (5) coming back opposite and at a distance from the pouch (40) in the absence of the spacer (9) of any thrust (F), the spacer (9) being decompressed, the sensor (3) being movable relative to the receptacle (21) between a first position in which the needle (5) is at a distance from and facing the pouch (40), the spacer (9) then being uncompressed, and a second position in which the pouch (40) is pierced by the needle (5) and in which the needle (5) is in contact with a volume of the pouch (40), the spacer (9) being compressed, the pouch (40) comprising a volume of a sensor preparation solution (3).

2. The assembly according to claim 1, comprising a rigid membrane (7a, 7b) fixed to the second face (91) of the spacer and extending away from and opposite the needle (5), said membrane (7a, 7b) being configured to close the aperture (12) to protect the needle (5).

3. The assembly according to claim 2, wherein the rigid membrane (7a, 7b) is formed from an elastic or semi-elastic material.

4. The assembly according to any one of the preceding claims, wherein the membrane (7a) is pierceable by the needle (5), the pouch (40) being arranged below the membrane (7a), the assembly being configured so that when the needle (5) undergoes a thrust (F) in the direction of the membrane (7a), the needle (5) pierces the membrane (7a) in addition to the pouch (40) and returns completely to the aperture (12) opposite and at a distance from the membrane (7a) in the absence of a thrust (F).

5. The assembly according to any one of claims 1 to 3, wherein the pouch (40) is arranged in the aperture (12) and rests on a surface (7b1) of the membrane (7b) which opens into the aperture (12).

6. The assembly according to any one of claims 1 to 5, wherein the spacer (9) in compressed or uncompressed position has a greater thickness or equal to 0.1 millimeter and less than or equal to 2 millimeters.

7. The assembly according to any one of claims 1 to 6, wherein the spacer (9) comprises at least a first spacer part (9a) and a second spacer part (9b) opposite the first part (9a), the first part (9a) and the second spacer part (9b) being joined together, the second part (9a) being preferably disjoint.

8. The assembly according to any one of claims 1 to 7 or claims 2 to 7, wherein the patch (4) comprises a peelable film (11) between an adhesive layer (8) of the patch (4) and the spacer (9), the first face (91) of the spacer (9) being fixedly bonded to the peelable film (11), the peelable film (11) being configured to enable the peelable film (11), the spacer (9), and optionally the membrane to be detached from an outer layer (41) of the patch (4).

9. The assembly according to any one of claims 1 to 8, comprising at least a first support element (212) located opposite the spacer (9), the first support element being configured to facilitate compression of the spacer (9) and piercing of the pouch (40) by the needle (5).

10. The assembly according to any one of claims 1 to 8, comprising at least one second support element (210, 213) located opposite the pouch (40), the second support element being configured to facilitate piercing of the pouch (40).
